# EUROPÄISCHE PATENTSCHRIFT

(11) **EP 1 359 901 B1**
(45) Veröffentlichungstag und Bekanntmachung des Hinweises auf die Patenterteilung: **26.11.2014**
(21) Anmeldenummer: 02716784.0
(22) Anmeldetag: 15.02.2002
(51) Int. Cl.: A61K 9/127

(54) **THERMOLABILES LIPOSOM MIT GEREGELTER FREIGABETEMPERATUR**
THERMOLABILE LIPOSOME WITH CONTROLLED RELEASE TEMPERATURE
LIPOSOME THERMOLABILE A TEMPERATURE DE LIBERATION REGULEE

(30) Priorität: 15.02.2001 DE 10107165
(43) Veröffentlichungstag der Anmeldung: 12.11.2003
(73) Patentinhaber: Max-Planck-Gesellschaft zur Förderung der Wissenschaften e.V., 80539 München (DE)
(72) Erfinder: EIBL, Hansjörg, 37120 Bovenden-Eddigehausen (DE); LINDNER, Lars, 81377 München (DE)
(74) Vertreter: Dey, Michael
(86) Internationale Anmeldenummer: PCT/EP2002/001653
(87) Internationale Veröffentlichungsnummer: WO 2002/064116

(56) Entgegenhaltungen:
- WO-A-99/52505
- DE-A- 19 622 224
- MARUYAMA K ET AL: "PHOSPHATIDYL POLYGLYCEROLS PROLONG LIPOSOME CIRCULATION IN VIVO" INTERNATIONAL JOURNAL OF PHARMACOGNOSY, SWETS & ZEITLINGER, LISSE, NL, Bd. 111, 1994, Seiten 103-107, XP000672277 ISSN: 0925-1618
- ZELLMER S ET AL: "THERMOLABILE LIPOSOMES WITH A HIGH FUSION EFFICACY AT 42C CAN BE MADE OF DIPALMITOYLPHOSPHATIDYLCHOLINE/FATTY ALCOHOL MIXTURES IN THE MOLAR RATIO OF 1/2" JOURNAL OF LIPOSOME RESEARCH, MARCEL DEKKER, NEW YORK, US, Bd. 4, Nr. 3, 1. November 1994 (1994-11-01), Seiten 1091-1113, XP000476608 ISSN: 0898-2104

## Beschreibung

Die Erfindung betrifft ein thermolabiles Liposom mit geregelter Freigabetemperatur für den Liposomeninhalt, insbesondere ein bei 37 °C in Serum stabiles Liposom mit einer geregelten Freigabetemperatur zwischen 40 und 80 °C.

Liposomen sind künstlich gebildete Vesikel aus Lipiddoppelschichten, welche ein wässriges Kompartiment einschließen (Bangham et al. 1965). Ursprünglich noch als Modellsystem für eine Zellmembran genutzt, wurden Liposomen in jüngster Zeit vor allem für den Arzneistofftransport weiterentwickelt. Liposomen können dabei die Verträglichkeit von Wirkstoffen steigern (Senkung der aktiven Toxizität von Amphothericin B durch liposomale Formulierung (AmBisome^{®}) um den Faktor 75 (Proffitt et al. 1991). Sie eröffnen aber auch die Möglichkeit, Arzneistoffe gezielt in erkranktes Gewebe zu transportieren (Forssen et al. 1992). Nach intravenöser Applikation werden Liposomen hauptsächlich in Zellen des retikuloendothelialen Systems (RES) der Leber und Milz aufgenommen (Gregoriadis und Nerunhun 1974). Um Liposomen als Arzneistoffträger für Zellen außerhalb des RES nutzen zu können, versuchte man die Zirkulationszeit der Liposomen im Blut zu erhöhen. Vor allem in Tumoren, die häufig sehr gut vaskularisiert sind (Jain 1996) und deren Gefäße durch geweitete interendotheliale Verbindungen, einer großen Anzahl von Fenestrierungen sowie diskontinuierlicher Basalmembranen (Murray and Carmichael 1995) besonders durchlässig sind, würde sich die Aufnahmewahrscheinlichkeit von Liposomen dadurch massiv erhöhen.

Maruyama et al., Int. J. Pharm., 111 (1994) 103-107, beschreibt Cholesterin-haltige Liposomen, deren Zirkulationszeit geringfügig durch Dipalmitoylphosphatidylpolyglycerine mit unterschiedlicher Kettenlänge beeinflusst werden kann. Die dort beschriebenen Liposomen weisen keine Phasenumwandlungstemperatur und keine Thermosensitivität auf.

DE 196 22 224 A1 beschreibt Phosphatidyloligoglycerine und mit diesen gebildete Liposomen. Die dort beschriebenen Liposomen weisen aufgrund des enthaltenen Cholesterins keine Phasenumwandlungstemperatur und somit keine Thermosensitivität auf.

Zellmer et al., J. Lip. Res. 4 (3) (1994) 1091-1113, beschreibt Liposomen, gebildet aus Dipalmitoylphosphatidylcholin, Dipalmitoylphosphatidylglycerin und verschiedenen langkettigen Fettalkoholen. Phosphatidyloligoglycerine werden nicht erwähnt.

WO 99/52505 beschreibt teilchenförmige Kontrastmittel für diagnostisches Imaging. Das teilchenförmige Material kann ein Membranmaterial enthalten, wie beispielsweise Phospholipide, welche Liposomen bilden. Phosphatidyloligoglycerine werden in WO 99/52505 nicht erwähnt.

Ein erstes Problem bei der Verwendung von Liposomen zum Transport von Wirkstoffen oder Markierungsstoffen in Körperflüssigkeiten liegt daher in der Erhöhung der Zirkulationszeit im Serum. Man hat zwar bereits gefunden, dass durch kovalente Bindung von Methoxypolyethylenglykolen an die Liposomenmembran die frühzeitige Erkennung der Liposomen durch das RES verhindert werden und damit die Zirkulationszeit verbessert werden kann. Neben einer Verbesserung der Zirkulationszeit besteht jedoch großes Interesse an einer Möglichkeit, durch Temperatureinwirkung eine gezielte Freigabe der Liposominhaltsstoffe bei bestimmter Temperatur zu erreichen.

Der Erfindung liegt daher die Aufgabe zugrunde, ein Liposom zu schaffen, welches eine wesentlich verbesserte Halbwertszeit im Serum aufweist, verglichen mit der üblichen Halbwertszeit bekannter Liposomen in der Größenordnung um 4 Stunden, und welches so beschaffen ist, dass der Inhalt der Liposomen bei einer bestimmten Temperatur rasch freigesetzt wird.

Gelöst wird diese Aufgabe gemäß der Erfindung durch ein Liposom mit geregelter Freigabetemperatur für den Liposominhalt, welches dadurch gekennzeichnet ist, dass es im Wesentlichen aus mindestens einem Phosphatidylcholin mit einer Hauptumwandlungstemperatur, die im Bereich von 0 bis 80 °C liegt und 2 bis 15 Gew.-% Phosphatidyloligoglycerin gebildet ist und frei von Cholesterin ist.

Erfindungsgemäß aufgebaute Liposomen weisen wesentlich verbesserte Halbwertszeiten von bis zu 20 Stunden im Serum auf und können durch geeignete Wahl der Komponenten und Mengen der Komponenten in Abhängigkeit von deren Hauptumwandlungstemperatur den oder die Inhaltsstoffe bei einer vorbestimmten vorgewählten Temperatur rasch und vollständig freigeben.

Bevorzugt ist das erfindungsgemäße Liposom aus 65 bis 75 % Dipalmitoyllecithin (1,2-Dipalmitoylglycero-3-phosphocholin), 10 bis 30 % Distearoyllecithin (1,2-Distearoylglycero-3-phosphocholin) und 5 bis 10 % Dipalmitoylphosphoglyceroglycerin zusammengesetzt. Diese bevorzugte Zusammensetzung ist bei 37 °C im Serum stabil, gibt jedoch den Inhalt bei Überschreiten einer Temperatur von 40 °C rasch frei.

Die vorstehend genannte bevorzugte Zusammensetzung des erfindungsgemäßen Liposoms lässt sich für andere Temperaturbereiche maßschneidern durch Wahl von Komponenten mit der jeweils geeigneten Hauptumwandlungstemperatur. In Tabelle 1 sind die Hauptumwandlungstemperaturen (T_{M}) von Phosphatidylcholinen angegeben, deren Hauptumwandlungstemperatur im Bereich von 0 bis 80 °C liegt. Die Hauptumwandlungstemperaturen sind wie aus der Tabelle erkennbar, abhängig von der Kettenlänge und der Verteilung über die Positionen 1 und 2 von Glycero-3-phosphocholin oder 1 und 3 von Glycero-2-phosphocholin.

**Tabelle 1**

| T_{M} | Phosphatidylcholin |
|---|---|
| 5 °C | 1-Palmitoyl-2-oleoyl- |
| 7 °C | 1-Stearoyl-2-oleoyl- |
| 11 °C | 1-Palmitoyl-2-lauroyl- |
| 14 °C | 1-Behenoyl-2-oleoyl- |
| 17 °C | 1-Stearoyl-2-lauroyl- |
| 19 °C | 1,3-Dimyristoyl- |
| 23 °C | 1,2-Dimyristoyl- |
| 27 °C | 1-Palmitoyl-2-myristoyl- |
| 33 °C | 1-Stearoyl-2-myristoyl- |
| 37 °C | 1-Myristoyl-2-palmitoyl- |
| 39 °C | 1,3-Dipalmitoyl- |
| 41 °C | 1,2-Dipalmitoyl- |
| 42 °C | 1-Myristoyl-2-stearoyl- |
| 46 °C | 1-Stearoyl-3-myristoyl- |
| 48 °C | 1-Stearoyl-2-palmitoyl- |
| 52 °C | 1-Palmitoyl-2-stearoyl- |
| 53 °C | 1,3-Distearoyl- |
| 56 °C | 1,2-Distearoyl- |
| 66 °C | 1,2-Diarachinoyl- |
| 75 °C | 1,2-Dibehenoyl- |
| 80 °C | 1,2-Dilignoceroyl- |

Die in Tabelle 1 aufgeführten Werte zeigen, dass durch Verwendung von Fettsäuren mit ungerader Kettenlänge und geeigneter Verteilung über das Glyceringrundgerüst praktisch jede gewünschte Temperatur im angegebenen Bereich von 0 bis 80 °C eingestellt werden kann.

Der Gehalt an Phosphatidyloligoglycerinen im erfindungsgemäßen Liposom ist essenziell für die erforderliche lange Zirkulationszeit im Serum. Phosphatidyloligoglycerine und ihre Herstellung sind bekannt aus der DE 196 22 224. Bevorzugt wird Dipalmitoylphosphoglyceroglycerin (DPPG2) verwendet.

Die erfindungsgemäßen thermolabilen Liposomen eignen sich hervorragend für die Anwendung auf verschiedenen Gebieten, insbesondere aber im Rahmen der regionalen Tiefenhyperthermie. Die regionale Tiefenhyperthermie, die in Kombination mit systemischer Chemotherapie an spezialisierten klinischen Zentren angewendet wird, bietet sich als ideale Technik für den tumorspezifischen liposomalen Transport und die anschließende Freisetzung eines Arzneistoffs aus der liposomalen Hülle an. So fördert die Hyperthermie zum einen die Extravasation von Liposomen aus Tumorkapillaren in das Interstitium (Gaber et al. 1996). Zum anderen kann durch die Erwärmung eine Freisetzung des Arzneistoffs aus speziellen thermosensitiven Liposomen induziert werden (Magin und Niesman 1984). Zusätzlich gibt es zahlreiche Hinweise für einen gesteigerten zytotoxischen Effekt von Zytostatika (Hahn et al. 1975) sowie einer Immunmodulation (Aktivierung von NK-Zellen; Multhoff et al. 1999) durch regionale Tiefenhyperthermie.

Die Thermolabilität der erfindungsgemäßen Liposomen wird durch die Phasenumwandlung der Phospholipide innerhalb der Liposomenmembran bedingt. Wird die Phasenumwandlungstemperatur durchlaufen, so kommt es einer kurzzeitigen Membraninstabilität und anschließende Freisetzung des liposomalen Inhalts.

Bei der oben erwähnten regionalen Hyperthermie wird der Tumor regional spezifisch überwärmt, sodass die Temperatur über der Grenztemperatur zur Freisetzung des Liposomeninhalts ansteigt. Als Liposomeninhalt kommen hierbei insbesondere in der Onkologie anwendbare Wirkstoffe wie z.B. Zytostatika in Betracht. Jedoch können auch Kontrastmittel, beispielsweise Gadolinium, Carboxyfluorescein o.dgl. allein oder zusammen mit einem Wirkstoff zur Freisetzung gebracht werden. Durch Verwendung von Kontrastmitteln wie Gadolinium wird eine nicht invasive Thermometrie möglich gemacht, bei der die erreichte Temperatur durch MRC bestimmt werden kann, die das freigesetzte Gadolinium misst. Bei dieser Anwendung der erfindungsgemäßen Liposomen wird zweckmäßig ein Hyperthermiegerät mit einem MRC-Gerät gekoppelt angewendet.

Eine weitere Anwendungsart für die erfindungsgemäßen Liposomen findet sich in der Augenheilkunde. Bei Einkapselung einer fluoreszierenden Markierungssubstanz lässt sich z.B. bei einer Laserbehandlung durch Freisetzung des fluoreszierenden Wirkstoffes wie z.B. Carboxyfluorescein nachweisen, wo die angestrebte Überwärmung tatsächlich aufgetreten ist.

Analog zu der am Auge erläuterten Einsatzmöglichkeit können daher erfindungsgemäße Liposomen generell dazu verwendet werden, erreichte Temperaturen nachträglich bestimmbar zu machen, z.B. wenn bestimmte Erhitzungstemperaturen o.dgl. festgestellt werden sollen.

Die erfindungsgemäßen Liposomen bestehen im Wesentlichen aus den oben angegebenen Substanzen, die bevorzugt in reiner Form vorliegen. Verunreinigungen sollten möglichst gering gehalten werden, insbesondere sollte ein möglichst geringer Cholesteringehalt vorliegen. Bevorzugt werden Liposomen, die völlig frei von Cholesterin sind da Cholesterin zu einer Verschmierung der Phasenumwandlungstemperatur führt und damit zu einem zu breiten thermischen Übergangsbereich.

Die Herstellung der erfindungsgemäßen thermolabilen Liposomen erfolgt in üblicher Weise durch Auflösen der Lipide, z.B. in Chloroform oder Chloroform/Wasser/Isopropanol,AbziehendesLösungsmittels,zweckmäßig im Vakuum im Rotationsverdampfer, Tempern der Lipide mit wässrigen Lösungen der einzukapselnden Inhaltsstoffe bei Temperaturen, die über der Phasenumwandlungstemperatur liegen. Die Dauer dieser Temperungsbehandlung beträgt zweckmäßig 30 bis 60 Minuten, kann jedoch aber auch kürzer oder länger sein. Durch mehrfach wiederholte Einfrier-Auftau-Vorgänge, beispielsweise 2- bis 5-faches Einfrieren und wieder Auftauen, erfolgt eine Homogenisierung. Schließlich wird die erhaltene Lipidsuspension durch eine Membran definierter Porengröße bei einer Temperatur über der Phasenumwandlungstemperatur extrudiert, um die angestrebte Liposomengröße zu erreichen. Als Membran eignen sich beispielsweise Polycarbonatmembranen definierter Porengröße, wie 100 bis 200 nm. Schließlich kann gegebenenfalls nicht eingekapselter Inhaltsstoff abgetrennt werden, beispielsweise durch Säulenchromatographie o.dgl.

Die folgenden Beispiele erläutern die Erfindung weiter.

### Beispiel 1

a) In der oben beschriebenen Weise werden Liposomen hergestellt, die aus 70 % DPPC, 20 % DSPC und 10 % DPPG2 zusammengesetzt sind. Sie enthalten eingekapseltes Carboxyfluorescein. Freies Carboxyfluorescein wurde vorher durch Säulenchromatographie mit Sephadex G75 abgetrennt.
b) Chamber Modell:
   Zur intravitalmikroskopischen Detektion der CF-Freisetzung aus thermolabilen Liposomen im Hyperthermiefeld eignet sich das Chambermodell (A-Mel-3-Melanom des syrischen Hamsters) des syrischen Hamsters. Hierbei werden einem syrischen Goldhamster eine transparente, dorsale Hautkammer implantiert. Nach Implantation der Hautkammer erfolgt die Implantation von Zellen des A-Mel-3-Melanoms des Hamsters auf das in der Kammer befindliche Subkutangewebe. Innerhalb von wenigen Tagen wächst innerhalb der Rückenhaut des Hamsters ein Tumor von mehreren Millimeter Größe. Die Mikrozirkulation sowie die Fluoreszenzanreicherung innerhalb des Tumors kann mit einem modifizierten Vitalmikroskop beobachtet werden. Die Tiere erhalten zusätzlich einen zentralen Venenkatheter. Mit Hilfe eines unter der Hautkammer befindlichen Wärmetauschers kann lokal eine Erwärmung des Tumors auf 42 °C erreicht werden. Die Tumortemperatur kann mit Hilfe einer Temperatursonde direkt gemessen werden (Endrich 1988).

Neben der Vitalmikroskopie ist auch das Verfahren der MRT-Messung am Chamber-Modell etabliert (Pahernik et al. 1999). Hierbei können analog zur Mikroskopie MRT-Bilder aufgenommen werden.

Die erhaltenen Werte in vitro zeigt Tabelle 2.

### A) in-vitro

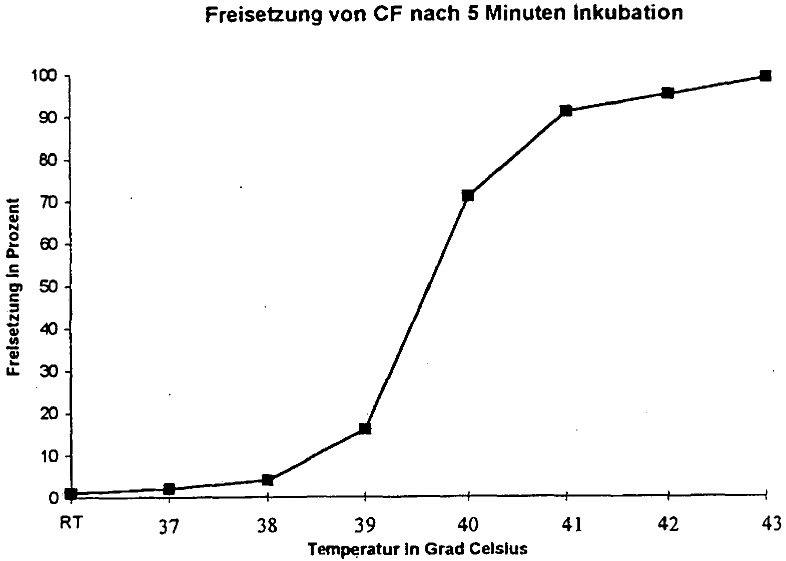

### Liposomenzusammensetzung:

DPPG:DSPC:DPPG-G2 = 7:2:1 Große Stabilität in Gegenwart von Serum bei 37°C (CF-Freisetzung nach 12 Stunden < 7%)

## Patentansprüche

1. Thermolabiles Liposom mit geregelter Freigabetemperatur für den Liposominhalt,
**dadurch gekennzeichnet,**
**dass** es im Wesentlichen aus mindestens einem Phosphatidylcholin mit einer Hauptumwandlungstemperatur, die im Bereich von 0 bis 80 °C liegt und 2 bis 15 Gew.-% Phosphatidyloligoglycerin gebildet ist und frei von Cholesterin ist.

2. Liposom nach Anspruch 1,
**dadurch gekennzeichnet,**
**dass** es mindestens ein Phosphatidylcholin, ausgewählt aus der Gruppe, bestehend aus 1-Palmitoyl-2-oleoylglycero-3-phosphocholin, 1-Stearoyl-2-oleoyl-3-phosphocholin, 1-Palmitoyl-2-lauroylglycero-3-phosphocholin, 1-Behenoyl-2-oleoylglycero-3-phosphocholin, 1-Stearoyl-2-lauroylglycero-3-phosphocholin, 1,3-Dimyristoylglycero-2-phosphocholin, 1,2-Dimyristoylglycero-3-phosphocholin, 1-Palmitoyl-2-myristoylglycero-3-phosphocholin, 1-Stearoyl-2-myristoylglycero-3-phosphocholin, 1-Myristoyl-2-palmitoylglycero-3-phosphocholin, 1,3-Pal mitoylglycero-2-phosphocholin, 1,2-Dipalmitoylglycero-3-phosphocholin, 1-Myristoyl-2-stearoylglycero-3-phosphocholin, 1-Stearoyl-3-myristoylglycero-2-phosphocholin, 1-Stearoyl-2-palmitoylglycero-3-phosphocholin, 1-Palmitoyl-2-stearoylglycero-3-phosphocholin, 1,3-Distearoylglycero-2-phoshocholin, 1,2-Distearoylglycero-3-phosphocholin, 1,2-Diarachihoylglycero-3-phosphocholin, 1,2-Dibehenoylglycero-3-phosphocholin und 1,2-Dilignoceroylglycero-3-phosphocholin, enthält.

3. Liposom nach Anspruch 1 oder 2,
**dadurch gekennzeichnet,**
**dass** es als Phosphatidyloligoglycerin Dipalmitoylphosphoglycero-glycerin enthält.

4. Liposom nach einem der vorhergehenden Ansprüche,
**dadurch gekennzeichnet,**
**dass** es im Wesentlichen aus 65 bis 75 % Dipalmitoyllecithin (DPPC), 15 bis 25 % Distearoyllecithin (DSPC) und 5 bis 10 % Dipalmitoylphosphoglyceroglycerin (DPPG2) besteht.

5. Liposom nach einem der vorhergehenden Ansprüche,
**dadurch gekennzeichnet,**
**dass** es einen Wirkstoff oder/und eine Markierungssubstanz enthält.

## Claims

1. A thermolabile liposome with controlled release temperature for the liposome contents, **characterized in that** it is essentially formed from at least one phosphatidylcholine having a main transition temperature which lies in the range from 0 to 80°C and 2 to 15% by weight of phosphatidyloligoglycerol and which is free from cholesterol.

2. The liposome as claimed in claim 1, **characterized in that** it contains at least one phosphatidylcholine selected from the group consisting of 1-palmitoyl-2-oleoylglycero-3-phosphocholine, 1-stearoyl-2-oleoyl-3-phosphocholine, 1-palmitoyl-2-lauroyl-glycero-3-phosphocholine, 1-behenoyl-2-oleoyl-glycero-3-phosphocholine, 1-stearoyl-2-lauroyl-glycero-3-phosphocholine, 1,3-dimyristoylglycero-2-phosphocholine, 1,2-dimyristoylglycero-3-phosphocholine, 1-palmitoyl-2-myristoylglycero-3-phosphocholine, 1-stearoyl-2-myristoylglycero-3-phosphocholine, 1-myristoyl-2-palmitoylglycero-3-phosphocholine, 1,3-palmitoylglycero-2-phosphocholine, 1,2-dipalmitoylglycero-3-phosphocholine, 1-myristoyl-2-stearoylglycero-3-phosphocholine, 1-stearoyl-3-myristoylglycero-2-phosphocholine, 1-stearoyl-2-palmitoylglycero-3-phosphocholine, 1-palmitoyl-2-stearoylglycero-3-phosphocholine, 1,3-distearoylglycero-2-phosphocholine, 1,2-di-stearoylglycero-3-phosphocholine, 1,2-di-arachinoylglycero-3-phosphocholine, 1,2-di-behenoylglycero-3-phosphocholine and 1,2-di-lignoceroylglycero-3-phosphocholine.

3. The liposome as claimed in claim 1 or 2, **characterized in that** it contains dipalmitoylphosphoglyceroglycerol as the phosphatidyloligoglycerol.

4. The liposome as claimed in one of the preceding claims, **characterized in that** it essentially consists of 65 to 75% of dipalmitoyllecithin (DPPC), 15 to 25% of distearoyllecithin (DSPC) and 5 to 10% of dipalmitoylphosphoglyceroglycerol (DPPG2).

5. The liposome as claimed in one of the preceding claims, **characterized in that** it contains an active compound or/and a labeling substance.

## Revendications

1. Liposome thermolabile avec température de libération réglée, **caractérisé en ce qu'**il est formé essentiellement d'au moins une phosphatidylcholine avec une température principale de transformation, qui se situe dans l'intervalle allant de 0 à 80°C, et de 2 à 15% en poids de phosphatidyloligoglycérine, et qu'il est exempt de cholestérol.

2. Liposome selon la revendication 1, **caractérisé en ce qu'**il contient au moins une phosphatidylcholine, choisie parmi le groupe consistant en la 1-palmitoyl-2-oléoylglycéro-3-phosphocholine, la 1-stéaroyl-2-oléoyl-3-phosphocholine, la 1-palmitoyl-2-lauroylglycéro-3-phosphocholine, la 1-béhénoyl-2-oléoylglycéro-3-phosphocholine, la 1-stéaroyl-2-lauroylglycéro-3-phosphocholine, la 1,3-dimyristoylglycéro-2-phosphocholine, la 1,2-dimyristoylglycéro-3-phosphocholine, la 1-palmitoyl-2-myristoylglycéro-3-phosphocholine, la 1-stéaroyl-2-myristoylglycéro-3-phosphocholine, la 1-myristoyl-2-palmitoylglycéro-3-phosphocholine, la 1,3-dipalmitoylglycéro-2-phosphocholine, la 1,2-di-palmitoylglycéro-3-phosphocholine, la 1-myristoyl-2-stéaroylglycéro-3-phosphocholine, la 1-stéaroyl-3-myristoylglycéro-2-phosphocholine, la 1-stéaroyl-2-palmitoylglycéro-3-phosphocholine, la 1-palmitoyl-2-stéaroylglycéro-3-phosphocholine, la 1,3-distéaroyl-glycéro-2-phosphocholine, la 1,2-distéaroylglycéro-3-phosphocholine, la 1,2-diarachinoylglycéro-3-phosphocholine, la 1,2-dibéhénoylglycéro-3-phosphocholine et la 1,2-dilignocéroylglycéro-3-phosphocholine.

3. Liposome selon la revendication 1 ou 2, **caractérisé en ce que** qu'il contient comme phosphatidyloligoglycérine, la dipalmitoylphospho-glycéroglycérine.

4. Liposome selon l'une des revendications précédentes, **caractérisé en ce qu'**il consiste essentiellement en 65 à 75% de dipalmitoyllécithine (DPPC), 15 à 25% de distéaroyllécithine (DSPC) et 5 à 10% de dipalmitoylphosphoglycéroglycérine (DPPG2).

5. Liposome selon l'une des revendications précédentes, **caractérisé en ce qu'**il contient un agent actif et/ou une substance de marquage.
